# EUROPÄISCHE PATENTSCHRIFT

(11) **EP 1 726 931 B1**
(45) Veröffentlichungstag und Bekanntmachung des Hinweises auf die Patenterteilung: **23.01.2008**
(21) Anmeldenummer: 06007554.6
(22) Anmeldetag: 11.04.2006
(51) Int. Cl.: G01K 1/10

(54) **Eintauchmesssonde**
Immersion probe
Sonde de mesure par immersion

(30) Priorität: 26.04.2005 DE 102005019666
(43) Veröffentlichungstag der Anmeldung: 29.11.2006
(73) Patentinhaber: Heraeus Electro-Nite International N.V., 3530 Houthalen (BE)
(72) Erfinder: Gerits, Erik, 3600 Genk (BE)
(74) Vertreter: Kühn, Hans-Christian

(56) Entgegenhaltungen:
- EP-A2- 0 322 201
- DE-C1- 19 849 433
- US-A- 2 999 121
- US-A- 3 455 164

## Beschreibung

Die Erfindung betrifft eine Eintauchmesssonde, insbesondere Einwurfmesssonde für Metallschmelzen mit einem Messkopf, an dem mindestens ein Sensorträger mit mindestens einem Sensor angeordnet ist, wobei der Sensorträger an oder in einer Öffnung des Messkopfes gehalten ist.

Derartige Messsonden sind beispielsweise aus DE 198 49 433 C1 bekannt. Einwurfmesssonden werden aus einer gewissen Höhe aus einer Halterung heraus in eine Metallschmelze geworfen. Der an einem Ende eines Trägerrohres gehalterte Messkopf ist in der Regel aus Stahl gebildet, um die nötige Masse zum Durchschlagen der auf einer Stahl- oder Eisenschmelze aufliegenden Schlackeschicht aufzuweisen. Innerhalb des Trägerrohres ist ein Signalkabel auf gewickelt, welches mit einer Messeinrichtung verbunden ist und welches sich während des Herabfallens in die Metallschmelze aus dem Trägerrohr heraus abwickelt. In dem Messkopf ist ein Sensorträger angeordnet mit mindestens einem Sensor, beispielsweise einem Temperatursensor oder einem elektrochemischen Element zum Messen des Sauerstoffgehalts der Metallschmelze. Beim Durchdringen der auf einer Stahlschmelze aufliegenden Schlackeschicht kann am Messkopf Schlacke haften bleiben. Diese am Messkopf, gegebenenfalls an der Schutzkappe für die Sensoren, anhaftende Schlacke kann die Messung der Eigenschaften der Stahlschmelze beeinflussen und zu Messfehlern führen

Aus US 3 455 164 ist ein üblicher Eintauchsensor bekannt, bei dem ein Messkopf aus gebundenem Gießereisand in einem Papprohr gehalten wird. Ein solcher Sensor wird an einer sogenannten Lanze gehalten und damit in eine Metallschmelze eingetaucht. Aus EP 322 201 A2 ist ein ähnlicher Eintauchsensor bekannt. Gemäß dieser Ausführung kann der Gießereisand durch Papprohre ersetzt werden. Zur Stabilität der Halterung der Sensorelemente während der Messung ist in der Druckschrift nichts offenbart.

Der Erfindung liegt die Aufgabe zugrunde, eine hinsichtlich der Messgenauigkeit und -zuverlässigkeit verbesserte Eintauchmesssonde bereit zu stellen.

Die Aufgabe wird durch eine Eintauchmesssonde gemäß Anspruch 1 gelöst. Vorteilhafte Ausgestaltungen sind in den Unteransprüchen angegeben.

Dadurch, dass an oder neben der Öffnung des Messkopfes ein verbrennbares und/oder ein Gas enthaltendes oder Gas bildendes poröses Material angeordnet ist, wird während des Eintauchens des aus Stahl gebildeten Messkopfes in die Schmelze oder in Schlacke durch die dabei erfolgende Erhitzung des Messkopfes Gas freigesetzt in unmittelbarer Umgebung des Sensorträgers, so dass das Anhaften von Schlacke verhindert wird oder bereits anhaftende Schlacke durch die explosionsartige Freisetzung von Gas wieder abgelöst wird. Das freigesetzte Gas entstammt den Poren des porösen Materials und wird durch Volumenausdehnung während der Erwärmung freigesetzt oder es entsteht zum Beispiel als Verbrennungsgas während des Verbrennens oder durch Verdampfen von Feuchtigkeit des entsprechenden Materials am Messkopf. Dazu ist es erforderlich, dass das während der Erhitzung des Messkopfes auf Temperaturen bis oberhalb 1.000°C gasfreisetzende Material (zum Beispiel durch Verbrennung, Verdampfung oder aus Gas enthaltenden Poren) möglichst dicht an dem Sensor angeordnet ist.

Insbesondere kann das poröse Material offenporig sein. Das verbrennbare Material besteht aus einem organischem Fasermaterial oder an oder neben der Öffnung des Messkopfes, in dem der Sensorträger gehaltert ist, ist ein bindemittelhaltiger Körper, insbesondere Gießereisand, angeordnet. Das verbrennbare oder ein Gas enthaltende Material kann auch Teil des Sensorträgers sein oder den Sensorträger selbst bilden. Beispielsweise kann der Sensorträger aus Gießereisand gebildet sein. Auch eine ring- oder teilringförmige Anordnung des verbrennbaren oder ein Gas enthaltenden Materials um den Sensorträger herum ist denkbar. Der Sensorträger kann vorzugsweise rastend mit dem Messkopf verbunden sein, um eine leichte Montage zu ermöglichen.

Nachfolgend wird ein Ausführungsbeispiel der Erfindung an Hand einer Zeichnung näher erläutert. In der Zeichnung zeigt:
- Fig. 1: einen Querschnitt durch eine Ausführungsform des Messkopfes sowie eine Draufsicht,
- Fig. 2: einen Querschnitt durch eine weitere Ausführungsform sowie eine entsprechende Draufsicht.

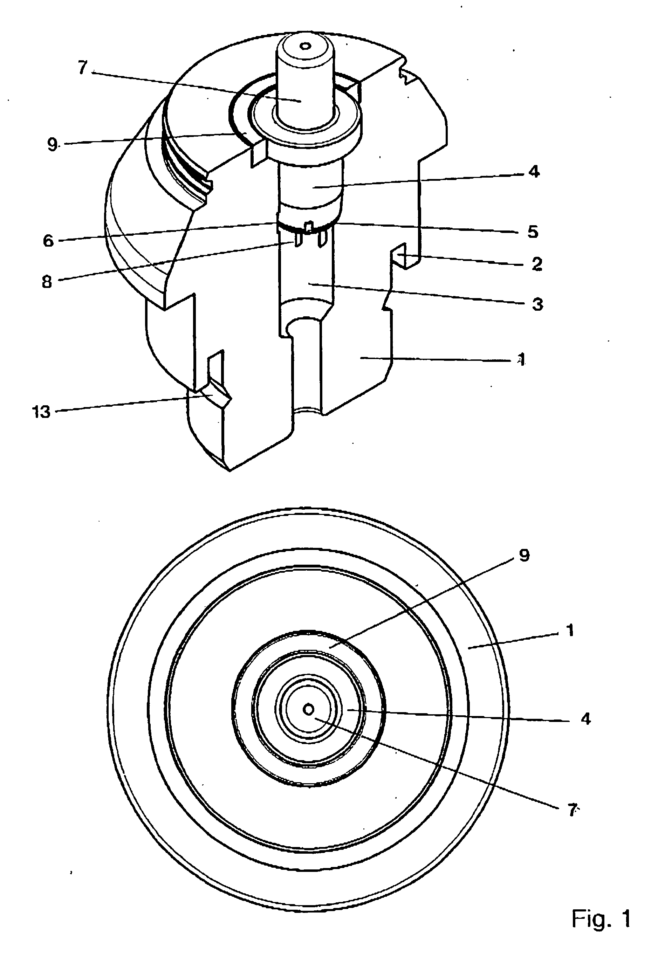

In Figur 1 ist der Messkopf 1 einer Einwurfmesssonde dargestellt. Der Messkopf 1 ist üblicherweise an einem Ende eines Trägerrohres, welches aus Pappe gebildet sein kann, gehaltert. Die Ringnut 2 oder die Ringnut 13 des Messkopfes 1 können dazu beitragen. Das Trägerrohr ist aus Gründen der Übersichtlichkeit in der Zeichnung nicht dargestellt.
Der Messkopf 1 besteht aus Stahl. Er weist eine zentrische Längsbohrung 3 auf. Im Eintauchende des Messkopfes 1 ist in der Bohrung 3 ein Sensorträger 4 gehaltert. Der Sensorträger 4 weist an seiner dem Eintauchende abgewandten Rückseite einen oder mehrere Widerhaken 5 auf, die beim Einschieben des Sensorträgers 4 in die Bohrung 3 in eine umlaufende Nut 6 einrasten und den Sensorträger 4 in der Bohrung 3 fixieren. Der Sensorträger 4 weist unter einer Schutzkappe 7 einen oder mehrere Sensoren auf, beispielsweise ein Thermoelement und/oder einen elektrochemischen Sensor zur Bestimmung eines Gasgehaltes der Stahlschmelze, zu deren Analyse die Einwurfmesssonde eingesetzt wird. Der Sensor ist über Kontakte 8 an der Rückseite des Sensorträgers 4 mit Signalleitungen verbunden, die durch die Bohrung hindurch mit einem Messgerät oder einem Computer verbunden sind. Am Eintauchende des Messkopfes 1 ist ein Ring 9 aus Gießereisand um den Sensorträger 4 herum angeordnet.

Bei Durchtauchen der auf der Stahlschmelze aufliegenden Schlackeschicht bzw. beim Eintauchen in die Stahlschmelze heizt sich der Messkopf sehr stark bis zur Temperatur der Schmelze auf. Dabei erfährt das in den Poren des Gießereisandes vorhandene Gas eine schnelle Volumenvergrößerung, so dass es sehr schnell aus den Ring 9 austritt und dabei eventuell an dem Sensorträger 4 oder der Schutzkappe 7 anhaftende Schlacke wegreißt. Dadurch beeinträchtigt die Schlacke die unmittelbare Umgebung des Sensors nicht, so dass die Messgenauigkeit erhöht wird.

Die Schutzkappe 7 kann aus Metall oder aus Pappe gebildet sein. Der Sensorträger 4 kann auch Stahl gebildet sein. Der Ring 9 kann statt aus Gießereisand auch beispielsweise aus Pappe gebildet sein. Der Messkopf kann prinzipiell auch ohne ein Trägerrohr verwendet werden.

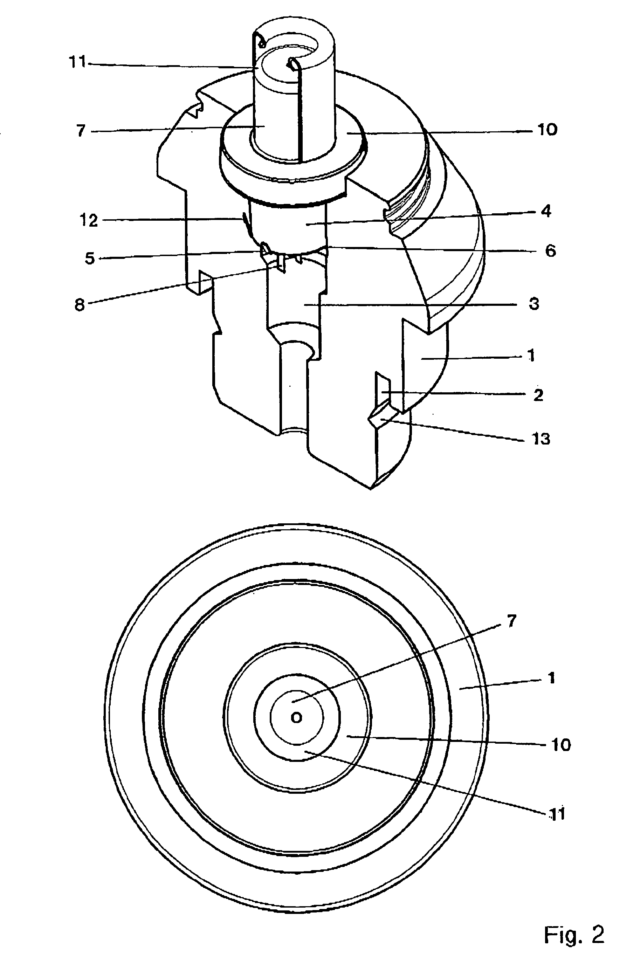

Figur 2 zeigt einen ähnlichen Messkopf 1. Der in der Bohrung 3 des Messkopfes 1 angeordnete Sensorträger 4 weist einen Ring 10 auf, der aus Gießereisand gebildet ist. Es ist auch möglich, den kompletten Sensorträger 4 aus Gießereisand auszubilden. Zumindest der Ring 10 kann auch aus Pappe oder einem ähnlichen verbrennbaren oder Gas enthaltenden porösen Material gebildet sein. Der Sensor des Sensorträgers 4 ist mit einem Schutzkappe 7 abdeckt, die wiederum mit einer Pappkappe 11 bedeckt ist. Sowohl die Pappkappe 11 als auch die Schutzkappe 7 werden beim Eintritt in die Stahlschmelze vernichtet, so dass der Sensor mit der Stahlschmelze in Berührung gelangt. Ein am Sensorträger 4 angeordnetes, fachübliches elektrochemisches Element benötigt zu seiner Funktionsfähigkeit einen sogenannten Badkontakt. Dieser Badkontakt wird zweckmäßigerweise dadurch hergestellt, dass der entsprechende, aus der Unterseite des Sensorträgers 4 herausgeführte Kontakt des elektrochemischen Elementes über ein Kontaktblech 12 oder einen anderen elektrischen, vorzugsweise metallischen Leiter direkt mit den metallischen Messkopf 1 elektrisch verbunden ist, so dass der Körper des Messkopfes 1 selbst den elektrischen Kontakt zur Metallschmelze herstellt.

## Patentansprüche

1. Eintauchmesssonde, insbesondere Einwurfmesssonde, für Metallschmelzen mit einem Messkopf (1), an dem mindestens ein Sensorträger mit mindestens einem Sensor angeordnet ist, wobei der Sensorträger (4) in einer Öffnung des Messkopfes gehalten ist, wobei der Messkopf aus Stahl gebildet ist und an oder direkt neben der Öffnung des Messkopfes ein verbrennbares und/oder ein Gas enthaltendes oder Gas bildendes poröses Material (9) angeordnet ist, welches als organisches Fasermaterial und/oder als bindemittelhaltiger Körper (9) ausgebildet ist.

2. Eintauchmesssonde nach Anspruch 1, **dadurch gekennzeichnet, dass** das poröse Material offenporig ist.

3. Eintauchmesssonde nach Anspruch 1 oder 2, **dadurch gekennzeichnet, dass** an oder neben der Öffnung Gießereisand angeordnet ist.

4. Eintauchmesssonde nach einem der Ansprüche 1 bis 3, **dadurch gekennzeichnet, dass** das verbrennbare oder ein Gas enthaltende Material mindestens einen Teil des Sensorträgers bildet.

5. Eintauchmesssonde nach einem der Ansprüche 1 bis 45, **dadurch gekennzeichnet, dass** der Sensorträger rastend mit dem Messkopf verbunden ist.

## Claims

1. Immersion measuring probe, in particular a drop-in measuring probe, for metal melts, comprising a measuring head (1), on which at least one sensor carrier with at least one sensor is arranged, wherein the sensor carrier (4) is held in an opening of the measuring head, wherein the measuring head is formed from steel and a porous material (9) which is combustible and/or contains a gas or forms a gas is arranged on or directly next to the opening of the measuring head, which porous material is configured as an organic fibre material and/or as a binder-containing body (9).

2. Immersion measuring probe according to claim 1, **characterised in that** the porous material is open-pored.

3. Immersion measuring probe according to claim 1 or 2, **characterised in that** foundry sand is arranged on or next to the opening.

4. Immersion measuring probe according to any one of claims 1 to 3, **characterised in that** the combustible material or material containing a gas forms at least a part of the sensor carrier.

5. Immersion measuring probe according to any one of claims 1 to 4, **characterised in that** the sensor carrier is in locking connection with the measuring head.

## Revendications

1. Sonde de mesure par immersion, notamment sonde de mesure par immersion largable, pour des bains de métal en fusion, comprenant une tête de mesure (1) sur laquelle est agencé au moins un support de capteur avec au moins un capteur, sonde dans laquelle le support de capteur (4) est maintenu dans une ouverture de la tête de mesure et la tête de mesure est réalisée en acier, et dans laquelle, au niveau de l'ouverture de la tête de mesure ou directement à côté de cette ouverture, est agencé un matériau (9) poreux, qui est combustible et/ou renferme un gaz ou produit un gaz, et est réalisé sous la forme d'un matériau fibreux organique et/ou sous la forme d'un corps (9) renfermant un liant.

2. Sonde de mesure par immersion selon la revendication 1, **caractérisée en ce que** le matériau poreux est à pores ouverts.

3. Sonde de mesure par immersion selon la revendication 1 ou 2, **caractérisée en ce qu'**au niveau ou à côté de l'ouverture est agencé du sable de fonderie.

4. Sonde de mesure par immersion selon l'une des revendications 1 à 3, **caractérisée en ce que** le matériau combustible ou renfermant un gaz forme au moins une partie du support de capteur.

5. Sonde de mesure par immersion selon l'une des revendications 1 à 4, **caractérisée en ce que** le support de capteur est relié ou assemblé par encliquetage à la tête de mesure.
